# EUROPÄISCHE PATENTSCHRIFT

(11) **EP 1 718 229 B1**
(45) Veröffentlichungstag und Bekanntmachung des Hinweises auf die Patenterteilung: **02.04.2008**
(21) Anmeldenummer: 04713506.6
(22) Anmeldetag: 23.02.2004
(51) Int. Cl.: A61B 17/86, A61B 17/74, A61B 17/68

(54) **KNOCHENSCHRAUBE**
BONE SCREW
VIS OSSEUSE

(43) Veröffentlichungstag der Anmeldung: 08.11.2006
(73) Patentinhaber: Synthes GmbH, 4436 Oberdorf (CH)
(72) Erfinder: SCHLIENGER, André, CH-4144 Arlesheim (CH); SENN Peter, CH-4437 Waldenburg (CH)
(74) Vertreter: Lusuardi, Werther
(86) Internationale Anmeldenummer: PCT/CH2004/000095
(87) Internationale Veröffentlichungsnummer: WO 2005/079685

(56) Entgegenhaltungen:
- EP-A- 0 441 577
- WO-A-01/50967
- DE-U- 20 305 713
- FR-A- 2 725 615
- GB-A- 2 307 179
- US-A1- 2002 147 454
- US-A1- 2003 187 447

## Beschreibung

Die Erfindung bezieht sich auf eine Vorrichtung zur Knochenfixation gemäss dem Oberbergriff des Patentanspruchs 1.

Eine solche Knochenschraube ist aus der DE-A 35 38 238 bekannt, welche zudem auch einen damit zusammenwirkenden Dübel zeigt. Allerdings wir bei dieser bekannten Vorrichtung der Dübel zuerst separat im Knochen verankert und dann die Knochenschraube in den Dübel eingedreht. Der Zweck dieser bekannten Vorrichtung ist somit die Verbindung zweier Knochenfragmente gemäss dem bereits aus der allgemeinen Befestigungstechnik bekannten Dübel/Schraubenprinzip.

Bei der Erfindung geht es primär nicht um eine Fixation zweier Knochenfragmente (erst sekundär wird diese Aufgabe mittels eines Marknagels realisiert), sondern um die stabile Verankerung einer Knochenschraube in der Querbohrung eines Marknagels. Die bekannten Querverriegelungsschrauben weisen nämlich alle ein Spiel zur Querbohrung des Marknagels auf. EP 0 441 577 offenbart so eine typische Intramedulläre Huftschaube mit ein Marknagel und eine Hülse.

Hier will die Erfindung Abhilfe schaffen. Der Erfindung liegt die Aufgabe zugrunde, eine Knochenschraube zu schaffen, welche spielfrei in die Querbohrung eines Marknagels einbringbar ist.

Die Erfindung löst die gestellte Aufgabe mit einer Vorrichtung, welche die Merkmale des Anspruchs 1 aufweist.

Die durch die Erfindung erreichten Vorteile liegen im wesentlichen in ihrer Einfachheit (vormontierte Hülse auf der Knochenschraube) und der damit erzielbaren Spielfreiheit zum Marknagel.

Bei einer besonderen Ausführungsform weist das Gewinde der Knochenschraube eine konstante Tiefe auf, wobei sich der Durchmesser des Kerns des Schaftes im Mittelbereich (L₄) in Richtung zum vorderen Ende von einem größeren Wert "k₂" zu einem kleineren Wert "k₁" verkleinert ohne den Wert Null anzunehmen. Die radiale Expansion erfolgt somit über die Erweiterung des Kerndurchmessers, was eine bestimmte Verklemmkraft in der Querbohrung des Marknagels garantiert. Die Erweiterung des Kerndurchmessers ergibt im Vergleich zu einer Erweiterung des Gewinde-Aussendurchmessers eine vergrösserte Masse, welche für die Blockierung der Schraube verschiebbar ist.

Bei einer speziellen Ausführungsform weist die Hülse ein Innengewinde auf. Durch die Steigung des Innengewindes wird die axiale Bewegung generiert, welche durch die bestimmte Geometrie des konischen Kernabschnitts im Mittelteil (L₄) die radiale Aufweitung der Hülse erzeugt. Die Dreh- bzw. Schraubbewegung wird für das Einbringen der Schraube in die Querbohrung eines Marknagels ohnehin benötigt.

Die Hülse kann einen oder mehrere Längsschlitze aufweisen. Dadurch ergibt sich der Vorteil, dass die radiale Aufweitung der Hülse mit weniger Kraftaufwand, bzw. mit einem geringeren Drehmoment erreicht werden kann. Die Vormontage der Hülse auf der Schraube ist einfacher, die Scherkräfte werden ausgeglichen und die Hülse steht unter geringerer Eigenspannung. Falls ein einziger Längsschlitz vorgesehen ist, kann dieser über die gesamte Höhe der Hülse verlaufen. Der Längsschlitz kann aber auch nur über einen Teil der Höhe der Hülse verlaufen, wobei er bei dieser Ausführung auf einer Seite offen oder beidseitig geschlossen sein kann. Im Falle solcher Teilschlitze können auch mehrere Längsschlitze vorhanden sein, vorzugsweise alternierend oben und unten offen.

Bei einer weiteren Ausführungsform, weist die Hülse mit dem Durchmesser D_{H} an ein einem ihrer Enden einen peripher umlaufenden Flansch mit vergrössertem Durchmesser D_{F} > D_{H} auf. Der Flansch dient als Anschlag, bzw. als Positionierung der Hülse am Marknagel, so dass sich die Knochenschraube mit ihrem sich konisch erweiternden Mittelteil durch die Hülse hindurchschrauben muss und diese radial expandiert, während dem die Hülse selbst ansteht.

Die Hülse kann beispielsweise aus einem nicht-resorbierbaren Kunststoff, vorzugsweise aus PEEK bestehen. Die Vorteile liegen in der Biokompatibilität, der guten Produzierbarkeit, der einfachen Montage und dem Umstand, dass beim Herausziehen des Nagels die Hülse zusammengedrückt werden kann, falls sie im Loch des Nagels hängen bleibt.

Die Hülse kann aber auch aus einem metallischen Werkstoff bestehen. Die Vorteile bestehen hier in der höheren Festigkeit, der Biokompatibilität und dem Umstand, dass die Hülse aus dem gleichen Material besteht wie der Marknagel und die Schraube. Die metallische Hülse ist aber auch duktiler, so dass sie etwas fliessen kann, falls sie schräg eingebracht wird. Schliesslich hat die metallische Hülse den Vorteil, dass keine Bruchstücke entstehen.

Die Hülse kann auch aus einem resorbierbaren Kunststoff bestehen. Ein erster Vorteil liegt darin, dass die winkelstabile Verriegelung mit der Zeit abnimmt. Ein zweiter Vorteil liegt darin begründet, dass beim allfälligen Verlieren einer solche Hülse kein grosser Nachteil entsteht, da sie mit der Zeit resorbiert.

Bei einer weiteren Ausführungsform ist zwischen dem hinteren Ende der Knochenschraube und demjenigen Teil des Schaftes mit grösserem Aussendurchmesser "d₂" ein Schaftteil im Bereich "L₂" mit einem vergrösserten Aussendurchmesser d₃ > d₂ angeordnet. Diese Anordnung hat den Vorteil, dass sie das in der vorderen Kortikalis des Knochens gebohrte Loch verschliesst, so dass der Flansch der Hülse ohne grossen Kraftaufwand durch das Loch in der ersten Kortikalis gebracht werden kann. Der Schaftteil kann dabei im Bereich "L₂" mit einem Aussendurchmesser d₃ > d₂ mit einem Aussengewinde versehen sein. Das Aussengewinde mit dem Aussendurchmesser d₃ ergibt wieder eine schraubengemässe Verankerung im Knochen.

Der mit den oben beschriebenen Knochenschrauben zu verwendende Marknagel weist einen Durchmesser D_{N} und eine quer zu seiner Längsachse verlaufende Querbohrung auf, welche zweckmässigerweise einen Durchmesser D_{B} aufweist, der sowohl grösser ist als d₂ als auch grösser ist als d_{B-L5}. Vorteilhafterweise gehorcht der Durchmesser D_{H} der Hülse der Bedingung D_{H}≤ 1,2 D_{B}. Die Länge H_{L} der Hülse sollte vorteilhafterweise kleiner als 1,5 D_{N} und grösser als 0,2 D_{N} sein.

Bei einer weiteren Ausführungsform weist der Marknagel eine durchgehende Kannulierung und eine Wandstärke W_{N} auf. Die Länge H_{L} der Hülse entspricht vorteilhafterweise der Wandstärke W_{N} des Marknagels.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Knochenschraube mit aufgesetzter Hülse;
Fig. 2 einen Längsschnitt durch die Knochenschraube nach Fig. 1 eingesetzt in einen Marknagel mit Querbohrung;
Fig. 3 einen Längsschnitt durch die Knochenschraube nach Fig. 1; und
Fig. 4 eine perspektivische Ansicht der Hülse nach Fig. 1.

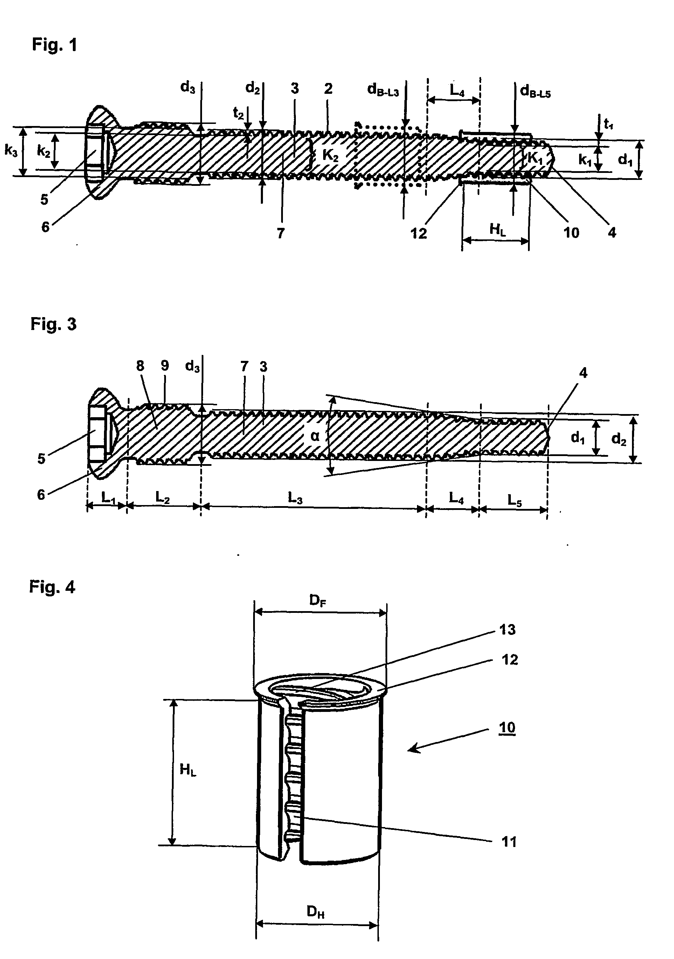

In den Fig. 1 und 3 ist eine Ausführungsform der Knochenschraube 1 dargestellt, welche einen Schaft 3, ein vorderes Ende 4 und ein hinteres Ende 5 mit einem Schraubenkopf 6 umfasst. Die Knochenschraube 1 hat fünf axial hintereinander angeordnete Segmente, wobei diese Segmente wie folgt ausgebildet sind:
- auf einer Länge L₁ vom hinteren Ende 5 gemessen erstreckt sich der Schraubenkopf 6;
- daran angrenzend auf einer Länge L₂ erstreckt sich ein Schaftteil 8 mit einem einen konstanten Aussendurchmesser d₃ und einen konstanten Kerndurchmesser k₃ aufweisenden Aussengewinde 9;
- daran angrenzend auf einer Länge L₃ ein Segment des Schaftes 3 mit einem einen konstanten Aussendurchmesser d₂ < k₃ und einen konstanten Kerndurchmesser k₂ aufweisenden Gewinde 2;
- daran angrenzend auf einer Länge L₄ ein sich mit dem Winkel α gegen das vordere Ende 4 verjüngendes Segment des Schaftes 3, so dass in diesem Segment der Aussendurchmesser d₂ des Gewindes auf einen Aussendurchmesser d₁ abnimmt. Ebenfalls verringert sich in diesem Segment der Kerndurchmesser k₂ auf einen Kerndurchmesser k₁; und
- zwischen dem Segment mit der Länge L₄ und dem vorderen Ende 4 erstreckt sich ein Segment des Schaftes 3 mit dem einen konstanten Aussendurchmesser d₁ und ebenfalls einen konstanten Kerndurchmesser k₁ aufweisenden Gewinde 2.

Das Gewinde 2 ist derart ausgestaltet, dass die Gewindetiefen t₁ und t₂ auf den Segmenten mit den Längen L₅ und L₃ gleich sind. Ferner ist in Fig. 1 die Hülse 10 vom vorderen Ende 4 soweit über den Schaft 3 geschraubt, bis ihr mit dem Flansch 12 versehenes Ende in das konische Segment mit der Länge L₄ reicht. Durch die Konizität wird die Hülse 10 leicht aufgeweitet und weist einen Durchmesser d_{B-L5} > D_{H} (Fig. 4) auf. Wird die Hülse 10 weiter gegen das hintere Ende 5 geschraubt (gestrichelt dargestellt) wird sie durch das konische Segment mit der Länge L₄ weiter aufgeweitet, bis sie auf dem einen konstanten Aussendurchmesser d₂ aufweisenden Segment mit der Länge L₃ einen Durchmesser d_{B-L3} erreicht.

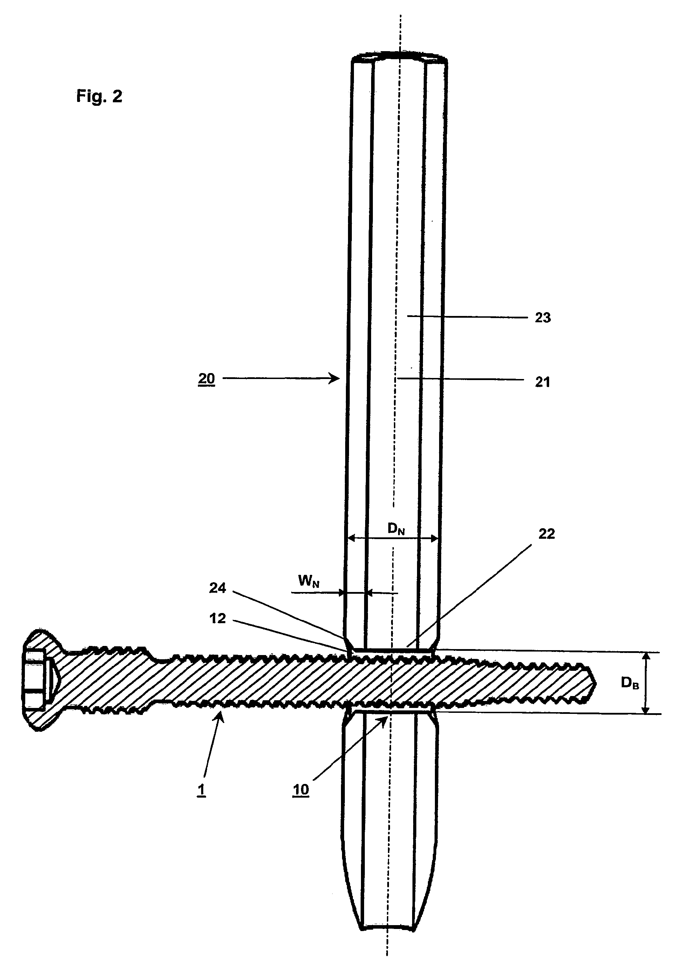

In Fig. 2 ist die in Fig. 1 dargestellte Ausführungsform der Knochenschraube 1 zusammen mit einer in eine Querbohrung 22 eines Marknagels 20 eingeführten Hülse 10 dargestellt. Der Marknagel 20 ist mit einer koaxial zu seiner Längsachse 21 durchgehenden Kannulierung 23 versehen und weist einen Aussendurchmesse D_{N} und eine Wandstärke W_{N} auf. Die Querbohrung 22 hat einen Durchmesser D_{B}, welcher ca. 90 % des Durchmessers d_{B-L3} der Hülse 10 beträgt. Die Hülse 10 ist vollständig in die Querbohrung 22 eingeführt und steht mit dem Flansch 12 auf der Wand einer Anschrägung 24 der Querbohrung 22 an.

In Fig. 4 ist eine Ausführungsform der radial expandierbaren Hülse 10 mit einem Durchmesser D_{H} dargestellt. Der Mantel der Hülse 10 ist mit einem durchgehenden Längsschlitz 11 ausgebildet, wodurch die Hülse 10 ihre Elastizität erhält. Ferner ist der Hohlraum der Hülse 10 mit einem Innengewinde 13 ausgestattet. Zur axialen Anlage an eine geeignete Auflagefläche ist an einem Ende der Hülse 10 ein Flansch 12 mit einem Durchmesser D_{F} > D_{H} angeordnet.

## Patentansprüche

1. Vorrichtung zur Knochenfixation umfassend
a) mindestens eine Knochenschraube (1) mit einem ein Gewinde (2) tragenden Schaft (3), einem vorderen Ende (4) und einem hinteren Ende (5), wobei sich der Aussendurchmesser "d" des Gewindes (2) oder der Kerndurchmesser "k" des Schaftes (3) in einem Mittelbereich (L₄) des Schaftes (3) von einer grösseren Dimension "d2", bzw. "k2", welche er auf einen Länge (L₃) aufweist, gegen das vordere Ende (4) hin zu einer kleineren Dimension "d1", bzw. "k1" verjüngt, wobei "d1", bzw. "k1" ungleich Null ist; und
b) einen Marknagel (20)
wobei, dass
c) der Schaft (3) mit einer konzentrisch angeordneten, radial expandierbaren Hülse (10) versehen ist, wobei
d) die Hülse (10) auf demjenigen Teil des Schaftes (3) positioniert ist, welcher den geringeren Aussendurchmesser "d1" aufweist; wobei
e) die Hülse (10) durch Schrauben in den Mittelbereich (L₄) auf einen Durchmesser d_{B-L5} und durch Schrauben auf die Länge (L₃) auf einen Durchmesser d_{B-L3} expandierbar ist;
f) der Marknagel (20) den Durchmesser D_{N} und eine quer zu seiner Längsachse (21) verlaufende Querbohrung (22) aufweist, welche einen Durchmesser D_{B} aufweist, der sowohl grösser ist als d₂ als auch grösser ist als d_{B-L5} aber kleiner als d_{B-L3}.

2. Vorrichtung nach Anspruch 1, **dadurch gekennzeichnet, dass** das Gewinde (2) eine konstante Tiefe aufweist und sich der Durchmesser des Kerns (7) des Schaftes (3) im Mittelbereich (L₄) in Richtung zum vorderen Ende (4) von einem größeren Wert "k₂" zu einem kleineren Wert "k₁" verkleinert ohne den Wert Null anzunehmen.

3. Vorrichtung nach Anspruch 1 oder 2, **dadurch gekennzeichnet, dass** die Hülse (10) ein Innengewinde (13) aufweist.

4. Vorrichtung nach einem der Ansprüche 1 bis 3, **dadurch gekennzeichnet, dass** die Hülse (10) mindestens einen Längsschlitz (11) aufweist.

5. Vorrichtung nach einem der Ansprüche 1 bis 4, **dadurch gekennzeichnet, dass** der Längsschlitz (11) über die gesamte Höhe der Hülse (10) verläuft.

6. Vorrichtung nach einem der Ansprüche 1 bis 4, **dadurch gekennzeichnet, dass** der Längsschlitz (11) nur über einen Teil der Höhe der Hülse (10) verläuft.

7. Vorrichtung nach Anspruch 6, **dadurch gekennzeichnet, dass** mehrere Längsschlitze (11) vorhanden sind, vorzugsweise alternierend oben und unten offen.

8. Vorrichtung nach einem der Ansprüche 1 bis 7, **dadurch gekennzeichnet, dass** die Hülse (10) mit dem Durchmesser D_{H} an ein einem ihrer Enden einen peripher umlaufenden Flansch (12) mit vergrössertem Durchmesser D_{F} > D_{H} aufweist.

9. Vorrichtung nach einem der Ansprüche 1 bis 8, **dadurch gekennzeichnet, dass** die Hülse (10) aus einem nicht-resorbierbaren Kunststoff, vorzugsweise aus PEEK besteht.

10. Vorrichtung nach einem der Ansprüche 1 bis 8, **dadurch gekennzeichnet, dass** die Hülse (10) aus einem metallischen Werkstoff besteht.

11. Vorrichtung nach einem der Ansprüche 1 bis 8, **dadurch gekennzeichnet, dass** die Hülse (10) aus bioresorbierbaren Kunststoff besteht.

12. Vorrichtung nach einem der Ansprüche 1 bis 11, **dadurch gekennzeichnet, dass** zwischen ihrem hinteren Ende 5 und demjenigen Teil des Schaftes (3) mit grösserem Aussendurchmesser "d₂" ein Schaftteil (8) im Bereich "L₂" mit einem vergrösserten Aussendurchmesser d₃ > d₂ angeordnet ist.

13. Vorrichtung nach Anspruch 12, **dadurch gekennzeichnet, dass** der Schaftteil (8) im Bereich "L₂" mit einem Aussendurchmesser d₃ > d₂ mit einem Aussengewinde (9) versehen ist.

14. Vorrichtung nach einem der Ansprüche 1 bis 13, **dadurch gekennzeichnet dass**
A) die Knochenschraube (1) einen Schraubenkopf (6) umfasst, welcher sich auf einer Länge (L₁) vom hinteren Ende (5) gemessen erstreckt;
B) auf einer Länge (L₃) ein Segment des Schaftes (3) mit einem einen konstanten Aussendurchmesser "d₂" und einen konstanten Kerndurchmesser "k₂" aufweisenden Gewinde (2) erstreckt;
C) daran angrenzend ein sich auf einer Länge (L₄) mit einem Winkel α gegen das vordere Ende (4) verjüngendes Segment des Schaftes (3) erstreckt, so dass in diesem Segment der Aussendurchmesser "d₂" des Gewindes auf einen Aussendurchmesser "d₁" abnimmt; und
D) auf einer Länge (L₅) zwischen dem Segment mit der Länge (L₄) und dem vorderen Ende (4) sich ein Segment des Schaftes (3) mit dem einen konstanten Aussendurchmesser "d₁" und ebenfalls einen konstanten Kerndurchmesser "k₁" aufweisenden Gewinde (2) erstreckt; wobei
E) Längen und Durchmesser dieser Segmente grösser Null sind.

15. Vorrichtung nach Anspruch 14, **dadurch gekennzeichnet, dass** im Segment des Schaftes (3) mit der Länge (L₄) der Kerndurchmesser "k₂" auf einen Kerndurchmesser "k₁" abnimmt

16. Vorrichtung nach Anspruch 14 oder 15, **dadurch gekennzeichnet dass** auf einer Länge (L₂), die an die Länge (L₁) angegrenzt ist, sich ein Schaftteil (8) mit einem einen konstanten Aussendurchmesser "d₃ > d₂" und einen konstanten Kerndurchmesser "k₃" aufweisenden Aussengewinde (9) erstreckt.

17. Vorrichtung nach einem der Ansprüche 14 bis 16, **dadurch gekennzeichnet, dass** die Gewindetiefen "t₁" und "t₂" auf den Segmenten mit den Längen (L₅) und (L₃) gleich sind.

18. Vorrichtung (1) nach einem der Ansprüche 1 bis 17, **dadurch gekennzeichnet, dass** der Durchmesser D_{H} der Hülse (10) der Bedingung D_{H}≤ 1,2 D_{B} gehorcht.

19. Vorrichtung (1) nach einem der Ansprüche 1 bis 18, **dadurch gekennzeichnet, dass** die Länge H_{L} der Hülse (10) kleiner als 1,5 D_{N} ist.

20. Vorrichtung (1) nach einem der Ansprüche 1 bis 19, **dadurch gekennzeichnet, dass** die Länge H_{L} der Hülse (10) grösser als 0,2 D_{N} ist.

21. Vorrichtung (1) nach einem der Ansprüche 1 bis 20, **dadurch gekennzeichnet, dass** der Marknagel (20) eine durchgehende Kannulierung 23 und eine Wandstärke W_{N} aufweist.

22. Vorrichtung (1) nach Anspruch 21, **dadurch gekennzeichnet, dass** die Länge H_{L} der Hülse (10) der Wandstärke W_{N} des Marknagels (20) entspricht.

## Claims

1. Device for bone fixation comprising
a) at least one bone screw (1) with a shaft (3) carrying a thread (2), a front end (4) and a rear end (5), the external diameter "d" of the thread (2) or the core diameter "k" of the shaft (3) tapering in a middle region (L₄) of the shaft (3) from a larger dimension "d2" or "k2" respectively which it exhibits on a length (L₃) towards the front end (4) to a smaller dimension "d1" or "k1" respectively, "d1" or "k1" not being equal to zero, and
b) a medullary pin (20), whereby
c) the shaft (3) is provided with a concentrically disposed, radially expandable sleeve (10),
d) the sleeve (10) being positioned on that part of the shaft (3), which has the smaller external diameter "d1",
e) the sleeve (10) is expandable to a diameter d_{B-L5} by screwing into the middle region (L₄) and to a diameter d_{B-L3} by screwing over the length (L₃),
f) the medullary pin (20) has the diameter D_{N} and a transverse borehole (22) extending transversely to its longitudinal axis (21), the transverse borehole having a diameter D_{B}, which is larger than d₂ as well as larger than d_{B-L5} but smaller than d_{B-L3}.

2. The device of claim 1, **characterized in that** the thread (2) has a constant depth and that the diameter of the core (7) of the shaft (3) decreases in the middle region (L₄) in the direction of the front end (4) from a larger value "k₂" to a smaller value "k₁" without assuming the value of zero.

3. The device of claims 1 on 2, **characterized in that** the sleeve (10) has an internal thread (13).

4. The device of one of the claims 1 to 3, **characterized in that** the sleeve (10) has at least one longitudinal slot (11).

5. The device of one of the claims 1 to 4, **characterized in that** the longitudinal slot (11) extends over the whole height of the sleeve (10).

6. The device of one of the claims 1 to 4, **characterized in that** the longitudinal slot (11) extends only over a portion of the height of the sleeve (10).

7. The device of claims 6, **characterized in that** several longitudinal slots (11) are present and preferably are open alternately at the top and at the bottom.

8. The device of one of the claims 1 to 7, **characterized in that** the sleeve (10) with the diameter D_{H} has a peripheral, encircling flange (12) with an enlarged diameter D_{F} > D_{H} at one of its ends.

9. The device of one of the claims 1 to 8, **characterized in that** the sleeve (10) consists of a non-absorbable plastic, preferably of PEEK.

10. The device of one of the claims 1 to 8, **characterized in that** the sleeve (10) consists of a metallic material.

11. The device of on the claims 1 to 8, **characterized in that** the sleeve (10) consists of a bio-absorbable plastic.

12. The device of one of the claims 1 to 11, **characterized in that** a shaft part (8) is disposed in the region "L₂" with an enlarged external diameter d₃ > d₂ between the end (5) of the bone screw (1) and that part of the shaft (3) with an enlarged diameter d₃ > d₂.

13. The device of claim 12, **characterized in that** the shaft part (8) is provided with an external diameter d₃ > d₂ with an external thread (9) in the region "L₂".

14. Device of one of the claims 1 to 13, **characterized in that**
A) the bone screw (1) comprises a screw head (6), which extends over a length (L₁), measured from the rear end (5);
B) a segment of the shaft (3), with a thread (2) having a constant external diameter "d₂" and a constant core diameter "k₂, extends over a length (L₃)
C) adjoining thereon, a segment of the shaft (3), tapering with an angle α towards the front end (4), extends over a length (L₄), so that the external diameter "d₂" of the thread decreases in this segment to an external diameter "d₁" and
D) on a length (L₅), between the segment with the length (L₄) and the front end (4), a segment of the shaft (3) extends with the thread (2) having a constant external diameter "d₁" and also a constant core diameter "k₁",
E) the lengths and diameters of these segments being larger than zero.

15. The device of claim 14, **characterized in that** the core diameter "k₂" decreases to a core diameter "k₁" in the segment of the shaft (3) having the length (L₄).

16. The device of claims 14 or 15, **characterized in that** a shaft portion (8) with a constant external diameter "d₃ > d₂" and an external thread (9) having a constant core diameter "k₃" extends on a length (L₂), which adjoins the length (L₁).

17. The device of one of the claims 14 to 16, **characterized in that** the depths of the threads "t₁" and "t₂" are identical on the segments with the length (L₅) and (L₃).

18. The device (1) of one of the claims 1 to 17, **characterized in that** the diameter D_{H} of the sleeve (10) fulfills the condition that D_{H} ≤ 1,2D_{B}.

19. The device (1) of one of the claims 1 to 18, **characterized in that** the length H_{L} of the sleeve (10) is more than 1,5D_{N}.

20. The device (1) of one of the claims 1 to 19, **characterized in that** the length H_{L} of the sleeve (10) is greater than 0,2D_{N}.

21. The device (1) of one of the claims 1 to 20, **characterized in that** the medullary pin (20) has a continuous cannulation (23) and a wall thickness W_{N}.

22. The device (1) of claim 21, **characterized in that** the length H_{L} of the sleeve (10) corresponds to the wall thickness W_{N} of the medullary pin (20).

## Revendications

1. Dispositif de fixation osseuse comprenant
a) au moins une vis à os (1) avec une tige (3) portant un filetage (2), une extrémité avant (4) et une extrémité arrière (5), dans laquelle le diamètre extérieur "d" du filetage (2) ou le diamètre du noyau "k" de la tige (3) rétrécit, dans une zone centrale (L₄) de la tige (3), vers l'extrémité avant (4), passant d'une dimension supérieure "d₂" ou "k₂", qu'elle présente sur une longueur (L₃), à une dimension inférieure "d₁" ou "k₁", "d₁" ou "k₁" n'étant pas égale à zéro ; et
b) un clou intramédullaire (20)
dans lequel
c) la tige (3) est pourvue d'une douille (10) radialement expansible disposée de manière concentrique, dans lequel
d) la douille (10) est positionnée sur la partie de la tige (3) qui présente le diamètre extérieur inférieur "d₁" ; dans lequel
e) la douille (10) peut être expansée à un diamètre d_{B-L5} par visser dans la zone centrale (L₄) et à un diamètre d_{B-L3} par visser sur la longueur (L₃),
f) le clou intramédullaire (20) présente le diamètre D_{N} et un alésage transversal (22) s'étendant transversalement à son axe longitudinal (21), lequel alésage présente un diamètre D_{B} qui est à la fois plus grand que d₂ et plus grand que d_{B-L5} mais plus petit que d_{B-L3}.

2. Dispositif selon la revendication 1, **caractérisé en ce que** le filetage (2) présente une profondeur constante et le diamètre du noyau (7) de la tige (3) rétrécit, dans la zone centrale (L₄) vers l'extrémité avant (4), passant d'une valeur supérieure "k₂" à une valeur inférieure "k₁", sans atteindre une valeur de zéro.

3. Dispositif selon la revendication 1 ou 2, **caractérisé en ce que** la douille (10) présente un taraudage (13).

4. Dispositif selon l'une quelconque des revendications 1 à 3, **caractérisé en ce que** la douille (10) présente au moins une fente longitudinale (11).

5. Dispositif selon l'une quelconque des revendications 1 à 4, **caractérisé en ce que** la fente longitudinale (11) s'étend sur toute la hauteur de la douille (10).

6. Dispositif selon l'une quelconque des revendications 1 à 4, **caractérisé en ce que** la fente longitudinale (11) ne s'étend que sur une partie de la hauteur de la douille (10).

7. Dispositif selon la revendication 6, **caractérisé en ce que** plusieurs fentes longitudinales (11) sont présentes, de préférence ouvertes de manière alternée en haut et en bas.

8. Dispositif selon l'une quelconque des revendications 1 à 7, **caractérisé en ce que** la douille (10) ayant le diamètre D_{H} présente, au niveau de l'une de ses extrémités, une collerette périphérique (12) présentant un diamètre plus grand D_{F} > DH.

9. Dispositif selon l'une quelconque des revendications 1 à 8, **caractérisé en ce que** la douille (10) est composée d'une matière plastique non résorbable, de préférence de PEEK.

10. Dispositif selon l'une quelconque des revendications 1 à 8, **caractérisé en ce que** la douille (10) est composée d'un matériau métallique.

11. Dispositif selon l'une quelconque des revendications 1 à 8, **caractérisé en ce que** la douille (10) est composée d'une matière plastique biorésorbable.

12. Dispositif selon l'une quelconque des revendications 1 à 11, **caractérisé en ce que**, entre son extrémité arrière (5) et la partie de la tige (3) présentant un diamètre extérieur supérieur "d₂", une partie de tige (8) dans la zone "L₂" est pourvue d'un diamètre extérieur plus grand d₃ > d₂.

13. Dispositif selon la revendication 12, **caractérisé en ce que** la partie de tige (8) dans la zone "L₂" est pourvue d'un diamètre extérieur d₃ > d₂ présentant un filetage (9).

14. Dispositif selon l'une quelconque des revendications 1 à 13,
**caractérisé en ce que**
A) la vis à os (1) comprend une tête de vis (6) qui s'étend sur une longueur (L₁) mesurée à partir de l'extrémité arrière (5) ;
B) un segment de la tige (3), avec un filetage (2) présentant un diamètre extérieur constant "d₂" et un diamètre du noyau constant "k₂", s'étend sur une longueur (L₃) ;
C) à la suite de celui-ci s'étend un segment de la tige (3) rétrécissant sur une longueur (L₄) à un angle α vers l'extrémité avant (4), de sorte que dans ce segment, le diamètre extérieur "d₂" du filetage diminue jusqu'à un diamètre extérieur "d₁" ; et
D) un segment de la tige (3), avec le filetage (2) présentant le diamètre extérieur constant "d₁" et également un diamètre du noyau constant "k₁", s'étend sur une longueur (L₅) entre le segment de longueur (L₄) et l'extrémité avant (4) ; dans lequel
E) les longueurs et diamètres de ces segments sont supérieurs à zéro.

15. Dispositif selon la revendication 14, **caractérisé en ce que**, dans le segment de la tige (3) de longueur (L₄), le diamètre du noyau "k₂" diminue jusqu'à un diamètre du noyau "k₁".

16. Dispositif selon la revendication 14 ou 15, **caractérisé en ce qu'**une partie de tige (8), avec un filetage (9) présentant un diamètre extérieur constant "d₃ > d₂" et un diamètre du noyau constant "k₃", s'étend sur une longueur (L₂) qui est contiguë à la longueur (L₁).

17. Dispositif selon l'une quelconque des revendications 14 à 16, **caractérisé en ce que**, sur les segments présentant les longueurs (L₅) et (L₃), les profondeurs de filetage "t₁" et "t₂" sont identiques.

18. Dispositif (1) selon l'une quelconque des revendications 1 à 17, **caractérisé en ce que** le diamètre D_{H} de la douille (10) remplit la condition D_{H} ≤ 1,2 D_{B}.

19. Dispositif (1) selon l'une quelconque des revendications 1 à 18, **caractérisé en ce que** la longueur H_{L} de la douille (10) est inférieure à 1,5 D_{N}.

20. Dispositif (1) selon l'une quelconque des revendications 1 à 19, **caractérisé en ce que** la longueur H_{L} de la douille (10) est supérieure à 0,2 DN.

21. Dispositif (1) selon l'une quelconque des revendications 1 à 20, **caractérisé en ce que** le clou intramédullaire (20) présente un canal traversant (23) et une épaisseur de paroi W_{N}.

22. Dispositif (1) selon la revendication 21, **caractérisé en ce que** la longueur H_{L} de la douille (10) correspond à l'épaisseur de paroi W_{N} du clou intramédullaire (20).
